## Europäisches Patentamt
## European Patent Office
## Office européen des brevets

(19)

(11) Veröffentlichungsnummer: **0 021 152**
**B1**

(12) # EUROPÄISCHE PATENTSCHRIFT

(45) Veröffentlichungstag der Patentschrift:
**15.02.84**

(51) Int. Cl.³: **G 01 N 33/54, C 07 G 7/00**

(21) Anmeldenummer: **80103095.8**

(22) Anmeldetag: **03.06.80**

(54) **Verfahren zur immunologischen Bestimmung von Basalmembranmaterial, hierfür geeignete Basalmembranfragmente und Verfahren zu deren Herstellung, bzw. Gewinnung.**

(30) Priorität: **11.06.79 DE 2923583**

(43) Veröffentlichungstag der Anmeldung:
**07.01.81 Patentblatt 81/1**

(45) Bekanntmachung des Hinweises auf die Patenterteilung:
**15.02.84 Patentblatt 84/7**

(84) Benannte Vertragsstaaten:
**AT BE CH DE FR GB IT LI LU NL SE**

(56) Entgegenhaltungen:
EUR. JOURNAL OF BIOCHEMISTRY, Band 102, Nr. 1, 1979, H. RODE et al.: "Immunochemical Characterization of the Basement Membrane Glycoprotein Laminin", Seiten 195-201
CHEMICAL ABSTRACTS, Band 90, Nr. 1, 1. Januar 1979, Zusammenfassung 4125a, Seiten 400-401 COLUMBUS OHIO (US), R. TIMPL et al.: "Chemical and immunological studies on basement membrane collagen from a murine tumor"
ANALYTICAL BIOCHEMISTRY, Band 104, Nr. 1, 1980, S.I. RENNARD et al.: "Enzyme-Linked Immunoassay (ELISA) for Connective Tissue Components", Seiten 205-214
THE JOURNAL OF BIOLOGICAL CHEMISTRY, Band 254, Nr. 19, 10. Oktober 1979, R. TIMPL et al.: "Laminin - A Glycoprotein from Basement Membranes", Seiten

(73) Patentinhaber: **Max-Planck-Gesellschaft zur Förderung der Wissenschaften e.V., Bunsenstrasse 10, D-3400 Göttingen (DE)**

(72) Erfinder: **Timpl, Rupert, Dr., Bergstrasse 18, D-8033 Kralling (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

(56) Entgegenhaltungen: (Fortsetzung)
9933-9937
CHEMICAL ABSTRACTS, Band 92, Nr. 25, 23. Juni 1980, Zusammenfassung Nr. 211289p, COLUMBUS OHIO (US), J. RISTELI et al.: "Radioimmunoassays for circulating basement membrane proteins: laminin and 7 S collagen"

## Verfahren zur immunologischen Bestimmung von Basalmembranmaterial, hierfür geeignete Basalmembranfragmente und Verfahren zu deren Herstellung bzw. Gewinnung

Die Erfindung betrifft ein Verfahren zur immunologischen Bestimmung von Basalmembranmaterial in Körperflüssigkeiten, hierfür geeignete neue Basalmembranfragmente und ihre Herstellung.

Es ist bekannt, dass es bei einer Vielzahl von zum Teil sehr häufigen Erkrankungen (Diabetes, Nephropathien, Sklerodermie, Leberfibrose, Vasculitis) zu einer Vermehrung von Basalmembranen besonders in den Gefässwänden kommt. Diese Gefässveränderungen stellen sehr häufig einen letalen Faktor dar. Durch Immunfluoreszenzuntersuchungen bei diesen Erkrankungen wurde nun gefunden, dass eine Basalmembranveränderung unter anderem einer erhöhten Produktion eines neuen, als Laminin bezeichneten Basalmembranbestandteils und von Basalmembrancollagen beruht. Histologische Untersuchungen gestatten zwar einen spezifischen Nachweis dieser Veränderungen, können aber nur an Biopsiematerial durchgeführt werden und lassen sich nicht quantitativ auswerten.

Bisher war es aus Biol. Chem. of Basement Membranes (Kefalides, ed) Seiten 215–228 Ac. Press, M.Y. nur bekannt, dass Antikörper gegen Basalmembrancollagen vom Typ IV erzeugt werden können, die spezifisch sind für diesen Collagentyp. Basalmembranveränderungen konnten damit aber nicht nachgewiesen werden.

Der Erfindung liegt daher die Aufgabe zugrunde, dieses Problem durch Schaffung eines quantitativen und spezifischen Verfahrens zur Bestimmung von Basalmembranantigenen im Blut und anderen Körperflüssigkeiten (Urin, Ascites) zu lösen. Eine weitere Aufgabe besteht darin, definierte Basalmembranantigene für dieses Verfahren zur Verfügung zu stellen.

Gelöst wird diese Aufgabe durch ein Verfahren zur immunologischen Bestimmung von Basalmembranmaterial in Körperflüssigkeiten, das dadurch gekennzeichnet ist, dass man markiertes Laminin, markiertes Lamininfragment P1 oder markiertes Basalmembranfragmente Col 1 (IV) als Antigen mit seinem spezifischen Antikörper in Gegenwart der zu bestimmenden Probe inkubiert, den gebildeten Antigen-Antikörper-Komplex abtrennt und die im Komplex oder in der abgetrennten Flüssigkeit enthaltene Menge an markiertem Antigen in an sich bekannter Weise bestimmt, wobei die Menge des im Komplex gebundenen markierten Antigens von der Menge des ungebundenen Antigens abhängig ist, so dass auf diese Weise der Gehalt an antigen wirksamem Basalmembranmaterial in der Körperflüssigkeit bestimmt werden kann.

Das erfindungsgemässe Verfahren beruht auf der Charakterisierung von drei neuen Strukturproteinen der Basalmembran, nämlich von Laminin, dem Lamininfragment Laminin P1 und dem Collagenpeptid Col 1 (IV). Diese drei neuen Substanzen, insbesondere aber die beiden Fragmente Laminin P1 und Col 1 (IV), zeigen eine hohe Stabilität gegen proteolytischen Abbau und gegen denaturierende Einflüsse und treten deshalb auch in gut nachweisbaren Mengen in Körperflüssigkeiten, insbesondere im Blut, auf, so dass sich durch ihre quantitative Bestimmung die Vermehrung von Basalmembranen feststellen lässt.

Bei Laminin handelt es sich um ein hochmolekulares Glykoprotein. Es ist dadurch gekennzeichnet, dass es ein Glykoprotein mit 12 bis 15 Gew.-% Kohlehydratgehalt und einem Molekulargewicht von 800 bis 1000 k darstellt und aus zwei Arten von disulfidverknüpften Polypeptidketten mit einem Molekulargewicht von etwa 220 und 440 k besteht und durch Extraktion von basalmembranhaltigem Gewebe in Gegenwart von Proteaseinhibitoren mit konzentrierten Salzlösungen, nachfolgende Extraktion des Gewebes mit 0,4 bis 0,6 M NaCl, Fällung aus letzterem Extrakt mit 3,3 bis 3,5 M NaCl und Chromatographie des aufgelösten Niederschlags erhältlich ist.

Durch Abbau von Laminin mit Protease, wie Pepsin, oder nach chemischen Methoden, beispielsweise durch Bromcyan, lässt sich daraus ein grosses Fragment gewinnen, welches als Laminin P1 bezeichnet wird. Dieses Fragment kann auch direkt aus Gewebe gewonnen werden.

Laminin P1 ist dadurch gekennzeichnet, dass es ein Molekulargewicht von 200 bis 300 k, einen Cysteingehalt von 12 bis 14 Mol.-%, hohe Affinität für Lectine und Beständigkeit gegen Collagenase aufweist und 1) durch Abbau von Laminin mit einer Protease oder Bromcyan oder 2) durch Extraktion basalmembranhaltiger Gewebe mit konzentrierten Salzlösungen, anschliessend mit 0,4 bis 0,6 M NaCl, jeweils in Gegenwart von Proteaseinhibitoren, Inkubation des verbleibenden Rückstandes mit einer Protease, Dialyse der erhaltenen Lösung, fraktionierte Fällung der erhaltenen Lösung zwischen 2 M und 4 M NaCl, Chromatographie über einen schwach basischen Kationenaustauscher und Chromatographie über Lectin erhältlich ist.

Typische Beispiele für Lectine, für welche Laminin P1 eine hohe Affinität aufweist, sind Concanavalin A und Weizenkeimlectin.

Das dritte neue Antigen, welches im Rahmen der Erfindung verwendet werden kann, ist ein Fragment aus einer disulfidreichen Variante von Basalmembrancollagen und ist sowohl gegen übliche Proteasen, wie Pepsin und Trypsin, als auch gegen bakterielle Collagenase (Cl-hystolyticum) resistent. Es wird als Col 1 (IV) bezeichnet und ist dadurch gekennzeichnet, dass es ein Molekulargewicht von 200 bis 300 k, einen Cysteingehalt von 2 bis 5 Mol.-%, ein Achsenverhältnis in der Grössenordnung 1 : 20, eine stabile Tripelhelix mit einem Schmelzpunkt im Bereich von 60 bis 70 °C aufweist, gegen Collagenase resistent ist und durch Extraktion basalmembranhaltiger Gewebe mit einer konzentrierten Salzlösung und anschliessend mit 0,4 bis 0,6 M NaCl, jeweils in Gegenwart von Proteaseinhibitoren, Inkubation des Extraktionsrückstandes mit einer Protease, Dialyse der erhaltenen Lösung, Fällung mit 2 M NaCl, Inkubation des wiederaufgelösten Präzipitats mit Collagenase, Dialyse und chromatographische Reinigung erhältlich ist.

Vergleichweise ist das Achsenverhältnis bei Collagen 1 : 200.

Die nachstehende Tabelle gibt die Aminosäurezusammensetzung der drei neuen Antigene an.

Tabelle

|      | Col 1 (IV) | Laminin | Laminin P 1 |
|------|-----------|---------|-------------|
| Hyp  | 90        | –       | –           |
| Asp  | 68        | 109     | 113         |
| Thr  | 25        | 58      | 56          |
| Ser  | 19        | 77      | 81          |
| Glu  | 85        | 122     | 106         |
| Pro  | 62        | 53      | 63          |
| Gly  | 323       | 93      | 118         |
| Ala  | 41        | 76      | 47          |
| Cys  | 45        | 30      | 136         |
| Val  | 28        | 48      | 40          |
| Met  | 4         | 14      | 6           |
| Ile  | 23        | 42      | 20          |
| Leu  | 51        | 92      | 54          |
| Tyr  | 13        | 27      | 28          |
| Phe  | 12        | 31      | 30          |
| His  | 15        | 24      | 33          |
| Hyl  | 39        | 2       | 2           |
| Lys  | 14        | 52      | 30          |
| Arg  | 43        | 50      | 38          |

Diese neuen Basalmembranbestandteile, die im folgenden als erfindungsgemässe Antigene bezeichnet werden, ermöglichen, wie bereits erwähnt, die erfindungsgemässe Bestimmung von Basalmembranmaterial in Körperflüssigkeiten, insbesondere im Blut, unter Anwendung der an sich bekannten immunologischen Nachweismethoden, welche auf der Konkurrenz einer bekannten Menge markierten Antigens mit einer unbekannten Menge des Antigens in der zu untersuchenden Probe um den gemeinsamen Antikörper beruhen. Es können dabei sowohl die bekannten Radio-Immun-Assay-(RIA)-Varianten als auch die Enzym-Immun-Assay-Varianten und analoge Bestimmungen unter Verwendung andersartiger Markierungen, beispielsweise Fluoreszenzmarkierung, Farbstoffmarkierung und dergleichen, angewendet werden. Derartige Methoden sind dem Fachmann bekannt und sollen hier nicht im einzelnen nochmals aufgeführt werden. Alle diese Verfahren beruhen darauf, dass man mit Hilfe möglichst hochgereinigter Antigene in geeigneten Versuchstieren ein Antiserum herstellt, dieses als solches oder die daraus isolierten spezifischen Antikörper gewinnt und gegebenenfalls nach Bindung der Antikörper bzw. Antiseren an einen festen Träger die übliche Antigen-Antikörper-Komplexbildungsreaktion durch Inkubation der Reaktionspartner miteinander ablaufen lässt. Je nach der Menge des in der zu untersuchenden Probe einer Körperflüssigkeit vorhandenen nichtmarkierten Antigens wird nur ein Teil des markierten Antigens in diesem Komplex gebunden und kann entweder durch Isolierung des Komplexes oder im Überstand gemessen werden. Da die Menge des im Komplex gebundenen markierten Antigens von der Menge des ungebundenen Antigens abhängig ist, kann auf diese Weise der Gehalt an antigenwirksamem Basalmembranmaterial in der Körperflüssigkeit bestimmt werden.

Die Herstellung der Antiseren kann in üblicher Weise durch subcutane Injektion an Versuchstieren, vorzugsweise Kaninchen, erfolgen. Zweckmässig wird dabei in Gegenwart des kompletten Freund'schen Adjuvans gearbeitet. Es können die in derartigen Fällen üblichen Antigenmengen verabreicht werden. Als besonders geeignete Dosis bei Verwendung von Kaninchen erwiesen sich 0,5 bis 1 mg/Tier. Das gebildete Antiserum wird dann in der dem Fachmann bekannten Weise gewonnen und kann als solches verwendet werden. Es ist auch möglich, den im Serum vorhandenen spezifischen Antikörper vorher noch zu reinigen, beispielsweise nach den Methoden der Affinitätschromatographie.

Die Markierung des Antigens kann mit den für die Markierung von Proteinen bekannten Methoden durchgeführt werden. Im Falle der radioaktiven Markierung mit einem Radionuclid wird als letzteres vorzugsweise Jod 125 verwendet. Die Markierung mit diesem Radionuclid kann dann nach der bekannten Chloramin T-Methode (Int. Arch. Allergy, 29, 185) erfolgen.

Eine bevorzugte Ausführungsform des erfindungsgemässen Verfahrens besteht darin, die Trennung des mit dem spezifischen Antiserum gebildeten Antigen-Antikörper-Komplexes von nichtgebundenem Antigen durch Verwendung eines zweiten Antikörpers durchzuführen. Bevorzugt wird hierbei als zweiter Antikörper ein Antikörper gegen Immunoglobulin G der für die Gewinnung des Antiserums verwendeten Tierart eingesetzt. Die Trennung des damit in unlösliche Form überführten Antigen-Antikörper-Komplexes von der Lösung kann nach hierfür üblichen Methoden erfolgen, wie Abzentrifugieren, Abfiltrieren und dergleichen. Alternativ wird vorzugsweise das Antiserum bzw. der Antikörper an einen festen Träger, beispielsweise die Innenwand eines Reagenzglases, gebunden.

Nach Abtrennung des Antigen-Antikörper-Komplexes wird, wie erwähnt, die Markierung, beispielsweise also die Radioaktivität oder die Enzymaktivität, die im Antigen-Antikörper-Komplex gebunden ist oder wahlweise auch im Überstand verblieben ist, bestimmt. Anhand einer Eichkurve, die mittels Proben von bekanntem Antigengehalt erstellt wurde, lässt sich dann die in der zu untersuchenden Probe enthaltene Menge an Antigen feststellen. Dabei ist prinzipiell die Menge des markierten Antigens, welches im gebildeten Antigen-Antikörper-Komplex gebunden ist, um so geringer, je mehr nichtmarkiertes Antigen in der zu bestimmenden Probe vorhanden ist.

Das erfindungsgemässe immunologische Bestimmungsverfahren erlaubt die Messung von Konzentrationen bis in den Bereich von 1 ng/ml. Damit ist es möglich, dieses Verfahren zur Feststellung von Basalmembranmaterial in tierischen und humanen Körperflüssigkeiten, insbesondere im Blut bzw. Serum, einzusetzen. Bei normalen Individuen liegt die Konzentration dieser Antigene im Serum im Bereich von 20 bis 50 ng/ml und erhöht sich bei Basalmembranveränderungen, wie sie z.B. beim experimentellen Diabetes beobachtet werden, signifikant. Mit Hilfe des erfindungsgemässen Verfahrens lassen sich derartige Veränderungen nunmehr relativ rasch und sicher feststellen.

Die im erfindungsgemässen Verfahren verwendeten neuen Antigene lassen sich prinzipiell aus allen Geweben gewinnen, die Basalmembranen enthalten. Bevorzugt wird Humanplacenta, da der Gehalt an zu gewinnendem Material dort deutlich grösser ist. Ebenfalls bevorzugt sind spezielle Tumorgewebe, welche grosse Mengen an Basalmembran produzieren, wie z.B. das EHS-Sarkom der Maus.

Bei der Gewinnung der erfindungsgemässen neuen Antigene wird auf jegliche Reindarstellung von Basalmembran aus diesen Geweben verzichtet. Die Gewebe werden zuerst mit hohen Salzkonzentrationen in Gegenwart von Proteaseinhibitoren extrahiert, wobei Begleitproteine entfernt werden. Bevorzugt wird hierbei 3,4 bis 4 M NaCl. Für die Extraktion wird das Gewebe natürlich zuerst in üblicher Weise zerkleinert bzw. homogenisiert. Nach der gegebenenfalls mehrmaligen Extraktion mit hoher Salzkonzentration erfolgt ein zweiter Extraktionsschritt mit niedriger Salzkonzentration, zweckmässig mit 0,4 bis 0,6 M NaCl, der ebenfalls mehrfach durchgeführt werden kann. Hierbei geht praktisch kein Basalmembrancollagen in Lösung, jedoch wird ein Teil des Laminins herausgelöst. Die so gewonnene Lösung kann zur Gewinnung des Laminins und des Lamininfragments Laminin P 1 verwendet werden, während der bei der doppelten Salzextraktion verbliebene unlösliche Geweberückstand als Ausgangsmaterial für die Gewinnung des Fragments Col 1 (IV) dient, aber auch zur Gewinnung von Laminin P 1 geeignet ist.

Als Proteaseinhibitoren werden beispielsweise Phenylmethylsulfonyl-fluorid, p-Chlormercuribenzoat oder Äthylen-diamintetraessigsäure verwendet. Auch andere Proteaseinhibitoren sind jedoch geeignet. Die Inhibitoren können einzeln oder gemischt verwendet werden. Geeignete Konzentrationen liegen gewöhnlich zwischen etwa 1 bis 50 mg/l.

Aus dem lamininhaltigen Extrakt lässt sich natives Laminin durch Salzfällung von Begleitproteinen trennen. Bevorzugt erfolgt die Fällung bei 3,3 bis 3,5 M NaCl.

Das so erhaltene Präzipitat wird wieder in Puffer aufgenommen und chromatographiert, beispielsweise an Agarose A1.5m. Man erhält so das oben beschriebene Laminin, welches in der beschriebenen Weise für das erfindungsgemässe Verfahren eingesetzt werden kann.

Aus dem so erhaltenen Laminin lässt sich das Fragment P1 durch Abbau mit einem proteolytischen Enzym oder nach chemischen Methoden, wie mit Bromcyan, gewinnen. Bevorzugt erfolgt der Abbau mit Pepsin in stark saurer Lösung von etwa pH 1,5 bis 2,5. Aus der so erhaltenen Lösung lässt sich durch fraktionierte Salzfällung das Fragmente P1 gewinnen. Die Fällung erfolgt vorzugsweise zuerst mit 2 M NaCl und anschliessend mit 4 M NaCl, wobei bei letzterem Schritt das Laminin P1 ausfällt.

Alternativ kann das Fragmente P1 direkt aus Basalmembranmaterial, welches nicht isoliert zu sein braucht, gewonnen werden. Bei dieser bevorzugten Gewinnungsmethode wird von dem unlöslichen Geweberückstand der zweistufigen Salzextraktion ausgegangen. Der Rückstand wird in Suspension gebracht und mit einem proteolytischen Enzym unter den für dieses Enzym geeigneten Temperatur- und pH-Wertbedingungen inkubiert. Bevorzugt verwendet man Pepsin und arbeitet bei einem pH-Wert von etwa 1,5 bis 2,5 bei Normaltemperatur oder geringfügig niedrigeren Temperaturen. Gut geeignet sind etwa 10 bis 20 °C. Danach wird vom Unlöslichen abgetrennt, niedermolekulares Material durch Dialyse entfernt und aus der erhaltenen Lösung durch Salzfraktionierung in der oben beschriebenen Weise das Laminin P1 gewonnen. Die Fällung erfolgt vorzugsweise zuerst mit 2 M NaCl, danach mit 4 M NaCl.

Aus der präzipitierten Fraktion 2 bis 4 M NaCl lässt sich das Laminin P1 durch Chromatographie weiter reinigen. Vorzugsweise wird zuerst eine Chromatographie an einem schwach basischen Kationenaustauscher, wie DEAE-Cellulose oder DEAE-Sephadex, durchgeführt. Eine Feinreinigung kann dann durch Bindung an in unlöslicher Form, z.B. durch Trägerbindung oder Vernetzung, vorliegendes Lectin und nachfolgende Elution mit einem geeigneten Kohlenhydrat erfolgen. Auch eine Gelfiltration, beispielsweise an vernetztem Dextran, wie Agarose A1.5M, kann zur Reinigung verwendet werden.

Zur Gewinnung des Fragments Col 1 (IV) wird ebenfalls von der Aufschlusslösung des zweistufig mit Salzlösungen extrahierten Gewebes ausgegangen. Das nach der Proteasenbehandlung erhaltene Präzipitat bis 2 M NaCl wird nach erneuter Auflösung mit Collagenase unter den für dieses Enzym geeigneten Temperatur- und pH-Wertbedingungen inkubiert, wobei alle übrigen Collagene, ausgenommen Col 1 (IV), abgebaut werden und durch Dialyse entfernt werden können. Die Feinreinigung kann durch Molekularsiebchromatographie biespielsweise an vernetztem Dextran oder/und Carboxymethylcellulose erfolgen.

Für die oben beschriebenen proteolytischen Verfahrensschritte wird zwar Pepsin bevorzugt, aber auch andere Proteasen, wie z.B. Trypsin, sind hierfür geeignet. Alternativ kann auch ein chemisches Abbauverfahren verwendet werden. Vorzugsweise erfolgt dies mit Bromcyan.

Die folgenden Beispiele erläutern die Erfindung weiter.

### Beispiel 1
### Herstellung des markierten Antigens

25 µg von Laminin P1 oder Peptid Col 1 (IV) werden mit 0,5 Milli-Curie Jod 125 nach der Chloramin-T-Methode markiert und ungebundenes Jod durch Dialyse oder Gelfiltration an Bio-Gel P-2 entfernt. Die weiteren Schritte werden vorzugsweise in Gegenwart von 0,04% eines nichtionischen Detergens, wie z.B. Tween 20, durchgeführt. Bindungskurven mit Antikörper werden mit 1 ng markiertem Peptid bestimmt.

### Durchführung der immunologischen Bestimmung (RIA)

Die Konzentration an Laminin P1 oder Col 1 (IV) in einer unbekannten Probe von Serum oder anderen Körperflüssigkeiten wird in folgendem Inhibitionstest bestimmt. Eine bestimmte Menge des spezifischen Antikörpers oder Antiserums wird mit der unbekannten Probe 16 Stunden bei 4°C vorinkubiert und nach Zugabe von 1 ng markiertem Antigen weitere 8 Stunden bei 4°C inkubiert. Danach wird ein Überschuss von Antikörper gegen Kaninchen-Immunoglobulin G zugesetzt und nach weiteren 16 Stunden bei 4°C das im Immunkomplex gebundene Antigen durch Zentrifugation abgetrennt. Die Inhibitionsaktivität der unbekannten Probe wird mit der Aktivität einer Standardkonzentration von nichtmarkiertem Antigen verglichen.

### Beispiel 2
### Herstellung von Laminin

Als Ausgangsmaterial werden Humanplacenta oder ein transplantierbarer Maustumor (EHS-Sarkom, beschrieben bei Orkin et al., J. Exp. Med. 145, 204–220 (1977) verwendet. Das Gewebe wird zwei- bis dreimal in einem 20fachen Überschuss an 3,4 M NaCl, 0,05 M Tris.HCl, pH 7,4 in Gegenwart der Proteaseinhibitoren Phenylmethylsulfonyl-fluorid (3 mg/l), p-Chlormercuribenzoat (3 mg/l) und ÄDTA (0,01 M) homogenisiert und extrahierbares Protein durch Zentrifugation entfernt. Der Rückstand wird dann zweimal mit 0,5 M NaCl, 0,05 Tris.HCl, pH 7,4, in Gegenwart der Proteaseinhibitoren über Nacht bei 4°C extrahiert. Der Extrakt enthält natives Laminin, das von begleitendem Protein durch Fällung mit 3,4 M NaCl und Chromatographie des wieder aufgelösten Präzipitats an Agarose A1.5m (1 M CaCl$_2$), 0,05 M Tris · HCl, pH 7,4, abgetrennt wird.

### Beispiel 3
### Herstellung des Peptids Laminin P1

Der nach der in Beispiel 2 beschriebenen Salzextraktionen verbleibende unlösliche Geweberückstand wird in 0,5 M Essigsäure homogenisiert (50 mg/g Trockengewicht), der pH-Wert durch Zugabe von HCl auf 2 adjustiert und die Suspension nach Zugabe von Pepsin (50 mg/g Trockengewicht) 24 Stunden bei 15°C inkubiert. Das enzymatisch aufgelöste Material wird durch Zentrifugation isoliert und bei 4°C gegen 0,5 M NaCl, 0,05 M Tris, pH 7,4, dialysiert. Aus dieser Lösung wird mit 2 M NaCl ein Gemisch collagener Proteine gefällt und durch nachfolgende Fällung des Überstandes mit 4 M NaCl das Fragment Laminin P1 angereichert. Das Präzipitat bis 2 M NaCl kann zur Gewinnung von Col 1 (IV) verwendet werden.

Laminin P1 wird aus dem Präzipitat (2 bis 4 M NaCl) an DEAE-Cellulose, die in 0,05 M Tris · HCl, pH 8,6, 2 M Urea äquilibriert ist und mit einem linearen NaCl-Gradienten (0 bis 0,4 M) eluiert wird, weiter gereinigt. Die Endreinigung erfolgt durch Bindung an eine Concanavalin A-Säule und Elution mittels 0,1 M α-Methylmannosid. Wenn nötig, kann durch Gelfiltration an Agarose A1 · 5m noch eine weitere Reinigung durchgeführt werden. Alternativ kann anstelle des Geweberückstandes auch Laminin, welches nach Beispiel 2 erhalten wird, eingesetzt werden.

### Beispiel 4
### Herstellung von Col 1 (IV) Zur Isolierung von Peptid Col 1 (IV) wird das nach Beispiel 3 erhaltene Gemisch collagener Proteine (Präzipitat 0,5 bis 2 M NaCl) in 0,05 M Tris · HCl, pH 7,4, 0,2 M NaCl, 0,002 M CaCl$_2$ (10 mg/ml) gelöst und nach Zugabe von bakterieller Collagenase (0,1 mg/ml) für 16 Stunden bei 20 oder 37°C inkubiert. Dabei werden mit Ausnahme des Peptids Col 1 (IV) alle übrigen Collagene zu niedermolekularen Peptiden angebaut, die durch Dialyse entfernt werden. Die weitere Reinigung erfolgt an Agarose A5M (1 M CaCl$_2$, 0,05 M Tris · HCl, pH 7,4) und anschliessende Bindung des Peptids an eine Säule von CM-Cellulose, die in 0,01 M Natriumacetat pH 4,0, 4 M Harnstoff äquilibriert wurde. Durch einen linearen NaCl-Gradienten (0 bis 0,2 M NaCl) wird reines Col 1 (IV) von der Säule eluiert.

**Patentansprüche**

1. Verfahren zur immunologischen Bestimmung von Basalmembranmaterial in Körperflüssigkeiten, dadurch gekennzeichnet, dass man markiertes Laminin, markiertes Lamininfragment P1 oder markiertes Basalmembranfragment Col 1 (IV) als Antigen mit seinem spezifischen Antikörper in Gegenwart der zu bestimmenden Probe inkubiert, den gebildeten Antigen-Antikörper-Komplex abtrennt und die im Komplex oder in der abgetrennten Flüssigkeit enthaltene Menge an markiertem Antigen in an sich bekannter Weise bestimmt, wobei die Menge des im Komplex gebundenen markierten Antigens von der Menge des ungebundenen Antigens abhängig ist, so dass auf diese Weise der Gehalt an antigen-wirksamem Basalmembranmaterial in der Körperflüssigkeit bestimmt werden kann.

2. Verfahren nach Anspruch 1, dadurch gekennzeichnet, dass man ein radioaktiv markiertes, enzymmarkiertes oder fluoreszenzmarkiertes Antigen verwendet.

3. Verfahren nach Anspruch 1 oder 2, dadurch gekennzeichnet, dass man zur Abtrennung des Antigen-Antikörper-Komplexes einen zweiten Antikörper zusetzt, dessen Antigen das Immunoglobulin G der für die Gewinnung des Antiserums verwendeten Tierart ist.

4. Verfahren nach Anspruch 1 oder 2, dadurch gekennzeichnet, dass das Antiserum bzw. der Antikörper an einen festen Träger gebunden werden.

5. Verfahren nach einem der vorhergehenden Ansprüche, dadurch gekennzeichnet, dass Kaninchenantiserum bzw. -antikörper verwendet wird.

6. Laminin, geeignet zur Durchführung des Verfahrens nach einem der Ansprüche 1 bis 5, dadurch gekennzeichnet, dass es ein Glycoprotein mit 12 bis 15 Gew.-% Kohlehydratgehalt und einem Molekulargewicht von 800 bis 1000 k darstellt und aus zwei Arten von disulfidverknüpften Polypeptidketten mit einem Molekulargewicht von etwa 220 bis 440 k besteht und durch Extraktion von basalmembranhaltigem Gewebe in Gegenwart von Proteaseinhibitoren mit konzentrierten Salzlösungen, nachfolgende Extraktion des Gewebes mit 0,4 bis 0,6 M NaCl, Fällung aus letzterem Extrakt mit 3,3 bis 3,5 M NaCl und Chromatographie des aufgelösten Niederschlags erhältlich ist und folgende Aminosäurezusammensetzung aufweist: ASP 109, THR 58, SER 77, GLU 122, PRO 53, GLY 93, ALA 76, CYS 30, VAL 48, MET 14, ILE 42, LEU 92, TYR 27, PHE 31, HIS 24, HYL 2, LYS 52, ARG 50.

7. Lamininfragment P1, welches für das Verfahren nach den Ansprüchen 1 bis 5 geeignet ist, dadurch gekennzeichnet, dass es ein Molekulargewicht von 200 bis 300 k, einen Cysteingehalt von 12 bis 14 Mol.-%, hohe Affinität für Lecitine und Beständigkeit gegen Collagenase aufweist und durch Abbau von Laminin mit einer Protease oder Bromcyan oder durch Extraktion basalmembranhaltiger Gewebe mit konzentrierten Salzlösungen, anschliessend mit 0,4 bis 0,6 M NaCl, jeweils in Gegenwart von Proteaseinhibitoren, Inkubation des verbleibenden Rückstandes mit einer Protease, Dialyse der erhaltenen Lösung, fraktionierte Fällung der erhaltenen Lösung mit 2 M und 4 M NaCl, Chromatographie über einen schwach basischen Kationenaustauscher und Chromatographie über Lectin erhältlich ist und folgende Aminosäurezusammensetzung hat: ASP 113, THR 56, SER 81, GLU 106, PRO 63, GLY 118, ALA 47, CYS 136, VAL 40, MET 6, ILE 20, LEU 54, TYR 28, PHE 30, HIS 33, HYL 2, LYS 30, ARG 38.

8. Basalmembranpeptid Col 1 (IV), geeignet für das Verfahren nach den Ansprüchen 1 bis 5, dadurch gekennzeichnet, dass es ein Molekulargewicht von 200 bis 300 k, einen Cysteingehalt von 2 bis 5 Mol.-%, ein Achsenverhältnis in der Grössenordnung 1 : 20, eine stabile Tripelhelix mit einem Schmelzpunkt im Bereich 60 bis 70 °C aufweist, gegen Collagenase resistent ist und durch Extraktion basalmembranhaltiger Gewebe mit einer konzentrierten Salzlösung und anschliessend mit 0,4 bis 0,6 M NaCl, jeweils in Gegenwart von Proteaseinhibitoren, Inkubation des Extraktionsrückstandes mit einer Protease, Dialyse der erhaltenen Lösung, Fällung mit 2 M NaCl, Inkubation des wiederaufgelösten Präzipitats mit Collagenase, Dialyse und chromatographische Reinigung erhältlich ist und folgende Aminosäurezusammensetzung hat: HYP 90, ASP 68, THR 25, SER 19, GLU 85, PRO 62, GLY 323, ALA 41, CYS 45, VAL 28, MET 4, ILE 23, LEU 51, TYR 13, PHE 12, HIS 15, HYL 39, LYS 14, ARG 43.

9. Verfahren zur Herstellung von Laminin, dadurch gekennzeichnet, dass ein basalmembranhaltiges Gewebematerial in Gegenwart von Proteaseinhibitoren zuerst mit hohen Salzkonzentrationen, dann mit 0,4 bis 0,6 M NaCl, jeweils wenigstens einmal extrahiert wird, Laminin aus dem mit 0,4 bis 0,6 M NaCl erhaltenen Extrakt durch Salzfällung abgetrennt und gegebenenfalls das erhaltene Präzipitat aufgelöst und chromatographiert wird.

10. Verfahren zur Gewinnung des Peptids Laminin P1, dadurch gekennzeichnet, dass man entweder den durch Extraktion basalmembranhaltiger Gewebe in Gegenwart von Proteaseinhibitoren mit konzentrierten Salzlösungen und anschliessend mit 0,4 bis 0,6 M NaCl erhaltenen unlöslichen Geweberückstand oder Laminin mit einer Protease inkubiert, die erhaltene Lösung dialysiert, aus der dialysierten Lösung die zwischen 2 M und 4 M NaCl ausfallende Fraktion gewinnt und über einen schwach basischen Kationenaustauscher und über Lectin chromatographiert.

11. Verfahren zur Herstellung des Basalmembranpeptids Col 1 (IV), dadurch gekennzeichnet, dass man den durch Extraktion basalmembranhaltiger Gewebe in Gegenwart von Proteaseinhibitoren mit konzentrierten Salzlösungen und anschliessend mit 0,4 bis 0,6 M NaCl erhaltenen unlöslichen Geweberückstand mit einer Protease inkubiert, die erhaltene Lösung dialysiert, aus der dialysierten Lösung das Col 1 (IV) mit 2 M NaCl fällt, die Fällung auflöst und mit Collagenase inkubiert, danach dialysiert und chromatographiert.

12. Verfahren nach einem der Ansprüche 9 bis 11, dadurch gekennzeichnet, dass man als basalmembranhaltiges Ausgangsmaterial Humanplacenta oder das EHS-Sarkom der Maus verwendet.

**Claims**

1. Process for the immunological determination of basal membrane material in body fluids, characterised in that one incubates marked laminine, marked laminine fragment P1 or marked basal membrane fragment Col 1 (IV) as antigen with its specific antibody in the presence of the sample to be determined, separates off the antigen-antibody complex formed and determines in per se known manner the amount of marked antigen present in the complex or in the separated liquid, whereby the amount of the marked antigen bound in the complex is dependent upon the amount of the non-bound antigen so that, in this way, the content of antigen-effective basal membrane material in the body fluid can be determined.

2. Process according to claim 1, characterised in that one uses a radioactively marked, enzyme marked or fluorescent marked antigen.

3. Process according to claim 1 or 2, characterised in that for the separation of the antigen-antibody complex, one adds a second antibody, the antigen of which is the immunoglobulin G of the kind of animal used for obtaining the antiserum.

4. Process according to claim 1 or 2, characterised in that the antiserum or the antibody is bound to a solid carrier.

5. Process according to one of the preceding claims, characterised in that rabbit antiserum or antibody is used.

6. Laminine suitable for the carrying out of the process according to one of claims 1 to 5, characterised in that it is a glycoprotein with 12 to 15 wt.% carbohydrate content and a molecular weight of 800 to 1000 k and consists of two types of disulphidelinked polypeptide chains with a molecular weight of about 220 to 440 k and is obtainable by extraction of basal membrane-containing tissue in the presence of protease inhibitors with concentrated salt solutions, subsequent extraction of the tissue with 0.4 to 0.6M NaCl, precipitation from the latter extract with 3.3 to 3.5M NaCl and chromatography of the dissolved precipitate and has the following amino acid composition: Asp 109, Thr 58, Ser 77, Glu 122, Pro 53, Gly 93, Ala 76, Cys 30, Val 48, Met 14, Ile 42, Leu 92, Tyr 27, Phe 31, His 24, Hyl 2, Lys 52, Arg 50.

7. Laminine fragment P1, which is suitable for the process according to claims 1 to 5, characterised in that it has a molecular weight of 200 to 300 k, a cysteine content of 12 to 14 mol%, high affinity for lecithins and resistance towards collagenase and is obtainable by the decomposition of laminine with a protease or cyanogen bromide or by extraction of basal membrane-containing tissue with concentrated salt solutions, subsequently with 0.4 to 0.6M NaCl, in each case in the presence of protease inhibitors, incubation of the remaining residue with a protease, dialysis of the solution obtained, fractional precipitation of the solution obtained with 2M to 4M NaCl, chromatography over a weakly basic cation exchanger and chromatography over lectine and has the following amino acid composition: Asp 113, Thr 56, Ser 81, Glu 106, Pro 63, Gly 118, Ala 47, Cys 136, Val 40, Met 6, Ile 20, Leu 54, Tyr 28, Phe 30, His 33, Hyl 2, Lys 30, Arg 38.

8. Basal membrane peptide Col 1 (IV), suitable for the process according to claims 1 to 5, characterised in that it has a molecular weight of 200 to 300 k, a cysteine content of 2 to 5 mol%, an axial ratio of the order of magnitude of 1 : 20, a stable triple helix with a melting point in the range of 60 to 70 °C, is resistant towards collagenase and is obtainable by extraction of basal membrane-containing tissue with a concentrated salt solution and subsequently with 0.4 to 0.6M NaCl, in each case in the presence of a protease inhibitor, incubation of the extraction residue with a protease, dialysis of the solution obtained, precipitation with 2M NaCl, incubation of the redissolved precipitate with collagenase, dialysis and chromatographic purification and has the following amino acid composition: Hyp 90, Asp 68, Thr 25, Ser 19, Glu 85, Pro 62, Gly 323, Ala 41, Cys 45, Val 28, Met 4, Ile 23, Leu 51, Tyr 13, Phe 12, His 15, Hyl 39, Lys 14, Arg 43.

9. Process for the preparation of laminine, characterised in that a basal membrane-containing tissue material is extracted, in each case at least once, in the presence of protease inhibitors first with high salt concentrations, then with 0.4 to 0.6M NaCl, laminine is separated from the extract obtained with 0.4 to 0,6M NaCl by salt precipitation and the precipitate obtained optionally dissolved and chromatographed.

10. Process for the obtaining for the peptide laminine P1, characterised in that one incubates either the insoluble tissue extract obtained by the extraction of basal membrane-containing tissue in the presence of protease inhibitors with concentrated salt solutions and subsequently with 0.4 to 0.6M NaCl or laminine with a protease, dialyses the solution obtained, obtains from the dialysed solution the fraction precipitating out between 2M and 4M NaCl and chromatographs over a weakly basic cation exchanger and over lectine.

11. Process for the preparation of the basal membrane peptide Col 1 (IV), characterised in that one incubates with a protease the insoluble tissue residue obtained by extraction of basal membrane-containing tissue in the presence of protease inhibitors with concentrated salt solutions and subsequently with 0.4 to 0.6M NaCl, dialyses the solution obtained, precipitates the Col 1 (IV) from the dialysed solution with 2M NaCl, dissolves the precipitate and incubates with collagenase, thereafter dialyses and chromatographs.

12. Process according to one of claims 9 to 11, characterised in that, as basal membrane-containing starting material, one uses human placenta or the EHS sarcoma of the mouse.

## Revendications

1. Procédé pour la détermination immunologique d'une matière de membrane basale dans les fluides du corps, caractérisé en ce que l'on incube de la laminine marquée, un fragment P1 de laminine marquée ou bien un fragment Col 1 (IV) de membrane basale marquée à titre d'antigène avec son anticorps spécifique, en présence de l'échantillon à analyser, que l'on sépare le complexe antigène-anticorps formé et que l'on détermine la quantité d'antigène marqué contenu dans le fluide séparé ou dans le complexe selon une méthode connue en elle-même, comme alors la quantité de l'antigène marqué lié dans le complexe dépend de la quantité d'antigène non lié, on peut déterminer de cette façon la teneur en matière de membrane basale active en tant qu'antigène dans le liquide corporel.

2. Procédé selon la revendication 1, caractérisé en ce que l'on utilise un antigène marqué par fluorescence, par une enzyme ou un élément radio-actif.

3. Procédé selon la revendication 1 ou 2, caractérisé en ce que pour séparer le complexe antigène-anticorps, on ajoute un deuxième anticorps, dont l'antigène est l'immunoglobuline G de l'espèce animale utilisée pour l'obtention de l'antisérum.

4. Procédé selon la revendication 1 ou 2, caractérisé en ce que l'antisérum ou encore l'anticorps est lié à un support solide.

5. Procédé selon l'une quelconque des revendications précédentes, caractérisé en ce que l'on utilise l'antisérum ou l'anticorps de lapin.

6. Laminine appropriée pour la réalisation du procédé selon l'une quelconque des revendications 1 à 5, caractérisée en ce qu'elle représente une glycoprotéine ayant 12 à 15% en poids d'hydrate de carbone et un poids moléculaire de 800 à 1000 k, qu'elle est constituée de deux sortes de chaînes polypeptidiques reliées par des ponts disulfure avec un poids moléculaire d'environ 220 et 440 k, et qu'elle est obtenue par extraction des tissus contenant des membranes basales en présence d'inhibiteurs de protéases avec des saumures concentrées, extraction suivante du tissu avec du NaCl 0,4 à 0,6 M, précipitation à partir du dernier extrait avec du NaCl 3,3 à 3,5 M et chromatographie du précipité dissous, et qu'elle présente la composition en acides aminés suivante: ASP 109, THR 58, SER 77, GLU 122, PRO 53, GLY 93, ALA 76, CYS 30, VAL 48, MET 14, ILE 42, LEU 92, TYR 47, PHE 31, HIS 24, HYL 2, LYS 52, ARG 50.

7. Fragment P1 de laminine qui est convenable pour le procédé selon l'une des revendications 1 à 5, caractérisé en ce qu'il présente un poids moléculaire de 200 à 300 k, une teneur en cystéine de 12 à 14 moles %, une affinité élevée pour la lécitine et une résistance à la collagénase, et qu'il est obtenu par décomposition de laminine avec une protéase ou du bromcyane, ou par extraction de tissus contenant des membranes basales avec des saumures concentrées, puis avec du NaCl 0,4 à 0,6 M, à chaque fois en présence d'inhibiteurs de protéases, incubation du résidu restant avec une protéase, dialyse de la solution obtenue, précipitation fractionnée de la solution obtenue avec du NaCl 2 et 4 M, chromatographie sur un échangeur de cations faiblement basique et chromatographie sur la lectine, et qui présente la composition d'acides aminés suivante: ASP 113, THR 66, SER 81, GLU 106, PRO 63, GLY 118, ALA 47, CYS 136, VAL 40, MET 6, ILE 20, LEU 54, TYR 28, PHE 30, HIS 33, HYL 2, LYS 30, ARG 38.

8. Peptide de membrane basale Col 1 (IV) convenable pour le procédé selon l'une des revendications 1 à 5, caractérisé en ce qu'il présente un poids moléculaire de 200 à 300 k, une teneur en cystéine de 2 à 5 moles %, un rapport des axes de l'ordre de grandeur de 1 : 20, une triple hélice stable avec un point de fusion dans la gamme de 60 à 70 °C, qu'il est résistant à la collagénase et qu'il peut être obtenu par extraction de tissus contenant des membranes basales avec une saumure concentrée et ensuite avec du NaCl 0,4 à 0,6 M, à chaque fois en présence d'inhibiteurs de protéases, incubation du résidu d'extraction avec une protéase, dialyse de la solution obtenue, précipitation avec du NaCl 2 M, incubation du précipité redissous avec la collagénase, dialyse et purification chromatographique, et qu'il présente la composition en acides aminés suivante: HYP 90, ASP 68, THR 25, SER 19, GLU 85, PRO 62, GLY 323, ALA 41, CYS 45, VAL 28, MET 4, ILE 23, LEU 51, TYR 13, PHE 12, HIS 15, HYL 39, LYS 14, ARG 43.

9. Procédé de préparation de laminine, caractérisé en ce que l'on extrait au moins une fois à chaque étape un tissu contenant des membranes basales en présence d'inhibiteurs de protéases d'abord avec de fortes concentrations salines, puis avec du NaCl 0,4 à 0,6 M, que l'on sépare la laminine à partir de l'extrait obtenu avec du NaCl 0,4 à 0,6 M par précipitation saline, et qu'éventuellement, on dissout le précipité obtenu et le chromatographie.

10. Procédé d'obtention du peptide laminine P1, caractérisé en ce que l'on incube soit le résidu insoluble obtenu par extraction de tissus contenant des membranes basales, en présence d'inhibiteurs de protéases avec des saumures concentrées, puis avec du NaCl 0,4 à 0,6 M, soit de la laminine avec une protéase, qu'on dialyse la solution résultante, qu'on recueille à partir de la solution dialysée la fraction précipitant avec du NaCl entre 2 et 4 M, et que l'on chromatographie sur un échangeur de cations faiblement basique et sur de la lectine.

11. Procédé de préparation de peptide de membrane basale Col 1 (IV), caractérisé en ce que l'on incube avec une protéase le résidu insoluble obtenu par extraction de tissus contenant des membranes basales en présence d'inhibiteurs de protéases avec des saumures concentrées, puis avec du NaCl 0,4 à 0,6 M, qu'on dialyse la solution obtenue, on fait précipiter le Col 1 (IV) avec du NaCl 2 M à partir de la solution dialysée, on dissout le précipité et on incube avec de la collagénase, puis on dialyse et chromatographie.

12. Procédé selon l'une des revendications 9 à 11, caractérisé en ce que l'on utilise à titre de matière première contenant des membranes basales du placenta humain ou le sarcome EHS de la souris.